# EUROPEAN PATENT APPLICATION

(11) **EP 2 653 534 A1**
(43) Date of publication of application: **23.10.2013**
(21) Application number: 11848080.5
(22) Date of filing: 07.12.2011
(51) Int. Cl.: C12N 5/071, C12Q 1/02, C12M 1/22

(54) **METHOD FOR FORMING CELL AGGREGATE**

(30) Priority: 13.12.2010 JP 2010277394; 02.12.2011 JP 2011265210
(71) Applicant: SHISEIDO CO., LTD., Chuo-ku Tokyo 104-8010 (JP)
(72) Inventor: FUJIWARA, Shigeyoshi, Yokohama-shi Kanagawa 224-8558 (JP); TAKEI, Keigo, Yokohama-shi Kanagawa 224-8558 (JP); SUDA, Yukimitsu, Tokyo 104-8010 (JP)
(74) Representative: Ulmann, Catherine Claire
(86) International application number: PCT/JP2011/078308
(87) International publication number: WO 2012/081470

(57) **Abstract**

A method for forming a cell aggregate in the present invention is a method for forming a cell aggregate by using a culturing vessel and culturing an adhesive cell, wherein the culturing vessel is such that an amino group, a carboxyl group, and/or a hydroxyl group, and a group represented by a general formula of 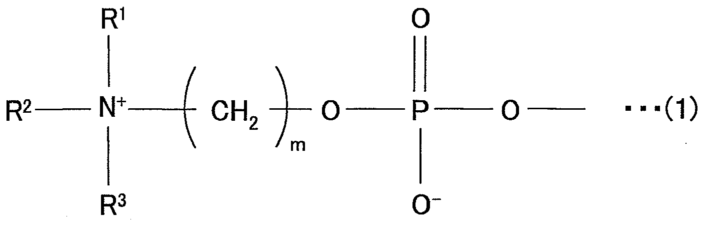 (in the formula, each of R¹, R², and R³ is independently an alkyl group with a carbon number greater than or equal to 1 and less than or equal to 6 and m is an integer greater than or equal to 2 and less than or equal to 6.) are present near a surface thereof and the adhesive cell is a mesenchymal stem cell, a hepatic cell, or a cancer cell.

## Description

### TECHNICAL FIELD

The present invention relates to a method for forming a cell aggregate, a method for screening a substance, a method for exploring a function of a cell, a cell aggregate, and a culturing vessel.

### BACKGROUND ART

Conventionally, an adhesive cell and a non-adhesive cell are cultured to form a cell aggregate.

Patent Document 1 discloses a method for forming a neural stem cell aggregate by suspending a tissue that includes a pluripotent neural stem cell in a medium that includes at least one kind of stem cell growth factor and conducting seeding and culturing thereof in a vessel for forming a neural stem cell aggregate. At that time, the vessel for forming a neural stem cell aggregate has a nonaqueous cured coating layer on an inner surface thereof and is manufactured by coating an inner face of a vessel with an aqueous resin to form an aqueous resin coating layer and subsequently curing the aqueous resin coating layer to be modified into a nonaqueous cured coating layer. Furthermore, a copolymer of 2-methacryloyloxyethylphosphorylcholine and another monomer (for example, butyl methacrylate, etc.) is illustrative for an aqueous resin.

However, there is a problem that even though such a vessel for forming a neural stem cell aggregate is used to culture an adhesive cell, a nonaqueous cured coating layer inhibits adhesion of an adhesive cell so that the adhesive cell is destroyed and it is not possible to form a cell aggregate of adhesive cells.

### PRIOR ART DOCUMENTS

### PATENT DOCUMENTS

Patent Document 1: Japanese Patent Application Publication No. 2008-220205

### SUMMARY OF THE INVENTION

### PROBLEMS TO BE SOLVED BY THE INVENTION

The present invention aims at providing a method for forming a cell aggregate capable of forming a cell aggregate of adhesive cells, a method for screening a substance and a method for exploring a function of a cell by using the method for forming a cell aggregate, a cell aggregate, and a culturing vessel, by taking a problem possessed by a conventional technique as mentioned above into consideration.

### MEANS FOR SOLVING THE PROBLEM

A method for forming a cell aggregate in the present invention is a method for forming cell aggregate by using a culturing vessel and culturing an adhesive cell, the culturing vessel is such that an amino group, a carboxyl group, and/or a hydroxyl group, and a group represented by a general formula of 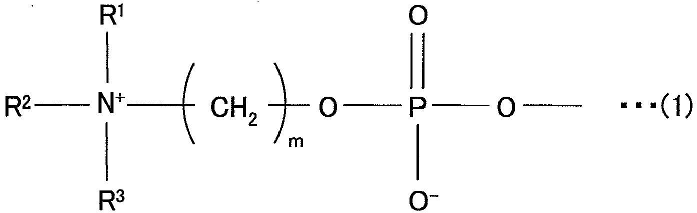 (in the formula, each of R¹, R², and R³ is independently an alkyl group with a carbon number greater than or equal to 1 and less than or equal to 6, and m is an integer greater than or equal to 2 and less than or equal to 6.) is present near a surface thereof and the adhesive cell is a mesenchymal stem cell, a hepatic cell, or a cancer cell.

A method for screening a substance in the present invention has a step of forming a cell aggregate by using a method for forming a cell aggregate in the present invention and a step of screening a substance by using the cell aggregate.

A method for exploring a function of a cell in the present invention has a step of forming a cell aggregate by using a method for forming a cell aggregate in the present invention and a step of exploring a function of a cell by using the cell aggregate.

A cell aggregate in the present invention is a cell aggregate wherein an adhesive cell is proliferated to aggregate autonomously and the adhesive cell is a mesenchymal stem cell, a hepatic cell, or a cancer cell.

A culturing vessel in the present invention is such that an amino group, a carboxyl group, and/or a hydroxyl group and a group represented by a general formula of 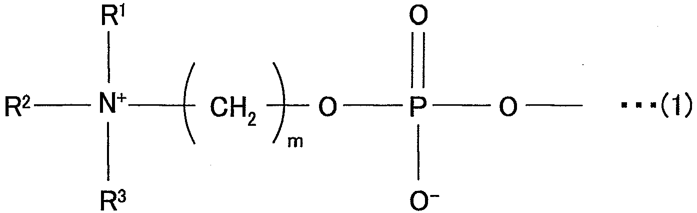 (in the formula, each of R¹, R², and R³ is independently an alkyl group with a carbon number greater than or equal to 1 and less than or equal to 6 and m is an integer greater than or equal to 2 and less than or equal to 6.) are present near a surface thereof.

### EFFECTS OF THE INVENTION

According to the present invention, it is possible to provide a method for forming a cell aggregate capable of forming a cell aggregate of adhesive cells, a method for screening a substance and a method for exploring a function of a cell by using the method for forming a cell aggregate, a cell aggregate, and a culturing vessel.

### BRIEF DESCRIPTION OF THE DRAWINGS

FIG. 1A is a diagram (part 1) illustrating one example of a method for forming a cell aggregate in the present invention.
FIG. 1B is a diagram (part 2) illustrating one example of a method for forming a cell aggregate in the present invention.
FIG. 2A is an optical micrograph illustrating a cell aggregate in Practical Example 1 (in a case where culturing was conducted for 2 days).
FIG. 2B is an optical micrograph illustrating a cell aggregate in Practical Example 1 (in a case where culturing was conducted for 5 days).
FIG. 3 is an optical micrograph illustrating a cultured cell in Comparative Example 1 (in a case where culturing was conducted for 5 days).
FIG. 4A is an optical micrograph illustrating a result of evaluation of an alkaline phosphatase activity of a cell aggregate in Practical Example 1.
FIG. 4B is an optical micrograph illustrating a result of evaluation of an alkaline phosphatase activity of a cultured cell in Comparative Example 1.
FIG. 5 is a fluorescence micrograph illustrating a result of evaluation (part 1) of immune double staining of a cell aggregate in Practical Example 1.
FIG. 6 is a fluorescence micrograph illustrating a result of evaluation (part 2) of immune double staining of a cell aggregate in Practical Example 1.
FIG. 7 is a fluorescence micrograph illustrating a result of evaluation (part 3) of immune double staining of a cell aggregate in Practical Example 1.
FIG. 8 is a fluorescence micrograph illustrating a result of evaluation (part 4) of immune double staining of a cell aggregate in Practical Example 1.
FIG. 9 is an optical micrograph illustrating a cell aggregate in Practical Example 2-1 (in a case where culturing was conducted for 2 days).
FIG. 10 is an optical micrograph illustrating a cell aggregate in Practical Example 2-2 (in a case where culturing was conducted for 7 days).
FIG. 11 is an optical micrograph illustrating a cell aggregate in Practical Example 2-3 (in a case where culturing was conducted for 7 days).
FIG. 12 is an optical micrograph illustrating a cultured cell in Comparative Example 2 (in a case where culturing was conducted for 7 days).
FIG. 13 is a photograph illustrating a result of evaluation of immunostaining of a cell aggregate in Practical Example 2-2.
FIG. 14 is an optical micrograph illustrating a cell aggregate in Practical Example 3 (in a case where culturing was conducted for 11 days).
FIG. 15 is an optical micrograph illustrating a cultured cell in Comparative Example 3 (in a case where culturing was conducted for 11 days).
FIG. 16 is a diagram illustrating a result of evaluation of an amount of a produced albumin in a cell aggregate in Practical Example 3 and a cultured cell in Comparative Example 3.
FIG. 17 is a diagram illustrating a result of evaluation of a CYP1A1 activity of a cell aggregate in Practical Example 3 and a cultured cell in Comparative Example 3.
FIG. 18 is a diagram illustrating a result of evaluation of immune expression in a cell aggregate in Practical Example 3 and a cultured cell in Comparative Example 3.
FIG. 19 is an optical micrograph illustrating a cell aggregate in Practical Example 4 (in a case where culturing was conducted for 8 days).
FIG. 20 is an optical micrograph illustrating a cultured cell in Comparative Example 4 (in a case where culturing was conducted for 8 days).
FIG. 21A is an optical micrograph illustrating a result of evaluation of an alkaline phosphatase activity of a cell aggregate in Practical Example 4.
FIG. 21B is an optical micrograph illustrating a result of evaluation of an alkaline phosphatase activity of a cultured cell in Comparative Example 4.
FIG. 22 is a diagram illustrating a result of evaluation of gene expression in a cell aggregate in Practical Example 4 and a cultured cell in Comparative Example 4.

### EMBODIMENTS FOR IMPLEMENTING THE INVENTION

Next, an embodiment for implementing the present invention will be described in conjunction with the drawings.

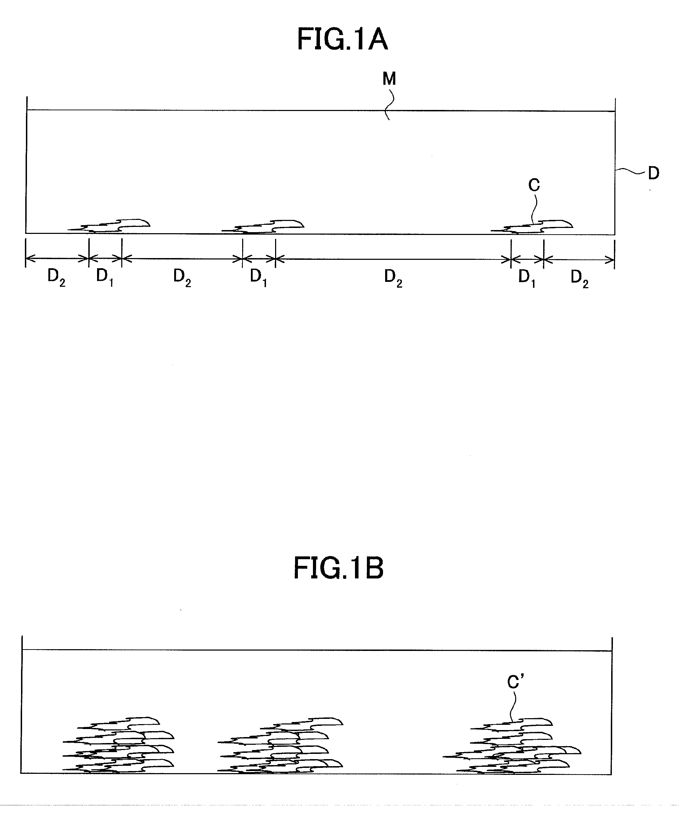

FIG. 1 illustrates one example of a method for forming a cell aggregate in the present invention.

A culturing vessel D has, on a bottom face thereof, an area D₁ wherein an amino group, a carboxyl group, and/or a hydroxyl group is/are present near a surface thereof and an area D₂ wherein a group represented by general formula (1) is present near a surface thereof.

As an adhesive cell C and a culture medium M are added to the culturing vessel D, the adhesive cell C adheres to a bottom face of the culturing vessel D (see FIG. 1A). It is considered that this is because the adhesive cell C adheres to the area D₁ wherein an amino group, a carboxyl group, and/or a hydroxyl group on a bottom face of the culturing vessel D is/are present near a surface thereof. Then, as the adhesive cell is cultured, the adhesive cell C proliferates and autonomously aggregates to form a cell aggregate C' (see FIG. 1B). It is considered that this is because the area D₂ wherein a group represented by general formula (1) on a bottom face of the culturing vessel D is present near a surface thereof suppresses adhesion of the adhesive cell C and accordingly a proliferated adhesive cell C is formed on the adhesive cell C. Hence, as the adhesive cell C is cultured by the culturing vessel D, it is possible for the adhesive cell C to proliferate and autonomously aggregate to form a cell aggregate C'.

The adhesive cell C is not particularly limited and it is possible to provide a mesenchymal cell, a hepatic cell, a hepatic stem cell, a myocardial cell, a cancer cell, a cancer stem cell, a fibroblast cell, a nerve cell, a vascular endothelial precursor cell, an epithelial cell, or the like.

A form of the culturing vessel D is not particularly limited and it is possible to provide a dish, a multi-well plate, a flask, a roller bottle, a unit of a device having a cell culturing process, or the like.

### [First embodiment of the culturing vessel D]

A first embodiment of the culturing vessel D is manufactured by reacting a surface modifying agent that has a group that has a reactivity with a carboxyl group, an amino group, or a hydroxyl group and a group represented by general formula (1), with a vessel wherein a carboxyl group, an amino group, and a hydroxyl group are present near a surface thereof. Herein, a molecular weight of a surface modifying agent is 225 - 650. Thereby, it is possible to introduce a group represented by general formula (1) at a high density.

A method for introducing an amino group near a surface of a vessel is not particularly limited and it is possible to provide a nitrogen plasma treatment, an ammonia plasma treatment, a method for reacting a surface treating agent therewith, a silicone gas phase treatment, or the like.

In a nitrogen plasma treatment, low temperature plasma is generated under a nitrogen gas atmosphere so that an amino group is introduced near a surface of a vessel (for example, see Surface and Coatings Technology 116 - 119 (1999) 802 - 807, Colloids and Surfaces A: Physicochem. Eng. Aspects 195 (2001) 81 - 95, Macromol. Chem. Phys. 200. 989 - 996 (1999)). Specifically, after a vessel is contained in a reaction vessel and an inside of the reaction vessel is vacuated by a vacuum pump, a nitrogen gas is introduced and glow discharge is conducted.

In an ammonia plasma treatment, low temperature plasma is generated under an ammonia gas atmosphere so that an amino group is introduced near a surface of a vessel. Specifically, after a vessel is contained in a reaction vessel and the reaction vessel is vacuated by a vacuum pump, an ammonia gas is introduced and glow discharge is conducted.

A material for composing a vessel is not particularly limited and it is possible to provide a poly(vinyl chloride), an acryl resin, a poly(styrene), a poly(propylene), a poly(ethylene), a polyester, a cycloolefin resin, a polycarbonate, a glass, or the like.

In a method for reacting a surface treating agent therewith, a surface treating agent such as an alkoxysilane, chlorosilane, or silazane that has an amino group is used to introduce an amino group near a surface of a vessel wherein a group capable of producing a silanol group through hydrolysis thereof, such as an alkokysilyl group, a silanol group, a hydroxyl group originating from a semimetal oxide, and/or a hydroxyl group originating from a metal oxide is/are present near a surface thereof. Specifically, first, a mixed liquid of water / 2-propanol is charged into a vessel, and after 3-aminopropyltrimethoxysilane is added thereto, heating is conducted at 100 °C to conduct reaction for 6 hours. Then, after cooling down to room temperature is conducted, washing with methanol and drying are conducted.

A material for composing a vessel is not particularly limited and it is possible to provide a 3-trimethoxysilylpropyl methacrylate - methyl methacrylate - divinylbenzene copolymer, polyvinyl chloride, an acryl resin, a poly(styrene), a poly(propylene), a poly(ethylene), polyester, a cycloolefin resin, a polycarbonate, a silica, a glass, an alumina, a talc, a clay, a mica, an asbestos, a titanium oxide, a zinc flower, an iron oxide, or the like.

In a silicone gas phase treatment, after a hydrosilyl group is introduced near a surface of a vessel by using 1, 3, 5, 7-tetramethylcyclotetrasiloxane, alkene that has an amino group is reacted therewith so that an amino group is introduced near a surface of a vessel (see, for example, Japanese Examined Patent Application Publication No. 1-54379, Japanese Examined Patent Application Publication No. 1-54380, and Japanese Examined Patent Application Publication No. 1-54381). Specifically, first, 1,3,5,7-tetramethylcyclotetrasiloxane and a vessel are put into a desiccator, and degassing is conducted by an aspirator. Then, after reaction is conducted at 80 °C for 16 hours, the vessel is taken therefrom and dried at 120 °C. Furthermore, after an obtained vessel is dipped in ethanol and allylamine is added thereto, a solution of chloroplatinic acid in ethanol is added thereto and stirring is conducted at 60 °C for 2 hours. After reaction is completed, washing with ethanol and drying under a reduced pressure are conducted.

A material for composing a vessel is not particularly limited and it is possible to provide a styrene - divinylbenzene copolymer, a poly(vinyl chloride), an acryl resin, a poly(styrene), a poly(propylene), a poly(ethylene), a polyester, a cycloolefin resin, a polycarbonate, a mica, a talc, a kaolin, alumina, a titanium oxide, a zinc oxide, an iron oxide, or the like.

An alkene that has an amino group is not limited to allylamine, and it is sufficient to be an amine that has a vinyl group, an amine that has an acryl group, or the like. Furthermore, an amino group may be protected with a butoxycarbonyl group, a benzyloxycarbonyl group, or the like. Moreover, an alkene that has a group capable of introducing an amino group by, for example, reaction with a diamine, such as an epoxy group, may be used instead of an alkene that has an amino group.

A method for introducing a carboxyl group and/or a hydroxyl group near a surface of a vessel is not particularly limited and it is possible to provide an oxygen plasma treatment, an ozone treatment, a water vapor plasma treatment, or the like.

In an oxygen plasma treatment, low temperature plasma is generated under an oxygen gas atmosphere, so that a carboxyl group and a hydroxyl group are introduced near a surface of a vessel. Specifically, after a vessel is contained in a reaction vessel and an inside of the reaction vessel is vacuated by a vacuum pump, an oxygen gas is introduced and glow discharge is conducted.

In an ozone treatment, an aqueous solution of ozone is charged into a vessel, so that a carboxyl group and a hydroxyl group are introduced near a surface of the vessel. Specifically, a 40 ppm aqueous solution of ozone is charged into a vessel and treated at room temperature for 15 minutes.

In a water vapor plasma treatment, low temperature plasma is generated under a water vapor atmosphere so that a hydroxyl group is introduced near a surface of a vessel. Specifically, after a vessel is contained in a reaction vessel and an inside of the reaction vessel is vacuated by a vacuum pump, water vapor is introduced and glow discharge is conducted.

A material for composing a vessel is not particularly limited and it is possible to provide a poly(styrene), a poly(ethyleneterephthalate), polycarbonate, polymethyl methacrylate, a poly(tetrfluoroethylene), a polyether ether ketone, a cycloolefin resin, polycarbonate, or the like.

A method for introducing a carboxyl group near a surface of a vessel is not particularly limited and it is possible to provide a method for reacting a surface treating agent therewith, a silicone gas phase treatment, or the like.

In a method for reacting a surface treating agent therewith, a surface treating agent such as an alkoxysilane, chlorosilane, or silazane that has a carboxyl group is used to introduce a carboxyl group near a surface of a vessel wherein a group capable of producing a silanol group through hydrolysis thereof, such as an alkoxysilyl group, a silanol group, a hydroxyl group originating from a semimetal oxide, and/or, a hydroxyl group originating from a metal oxide is present near a surface thereof. Specifically, first, triethoxysilylpropylsuccinic anhydride is dissolved in N,N-dimethylformamide, distilled water and 4-dimethylaminopyridine are added thereto, and stirring is conducted at room temperature for 16 hours to synthesize a silane coupling agent that has a carboxyl group. Then, after a mixed liquid of water / 2-propanol is charged into a vessel and a silane coupling agent that has a carboxyl group is added thereto, heating at 100 °C is conducted to conduct reaction for 6 hours. Furthermore, after cooling down to room temperature is conducted, washing with methanol and drying are conducted.

A material for composing a vessel is not particularly limited and it is possible to provide a 3-trimethoxysilylpropyl methacrylate - methyl methacrylate - divinylbenzene copolymer, a silica, a glass, an alumina, a talc, a clay, a mica, an asbestos, titanium oxide, zinc flower, iron oxide, or the like.

In a silicone gas phase treatment, after 1,3,5,7-tetramethylcyclotetrasiloxane is used to introduce a hydrosilyl group near a surface of a vessel, an alkene that has a carboxyl group is reacted therewith so that a carboxyl group is introduced near a surface of the vessel (see, for example, Japanese Examined Patent Application Publication No. 1-54379, Japanese Examined Patent Application Publication No. 1-54380, and Japanese Examined Patent Application Publication No. 1-54381). Specifically, first, a vessel charged with 1,3,5,7-tetramethylcyclotetrasiloxane is put into a desiccator and degassing is conducted by an aspirator. Then, after reaction is conducted at 80 °C for 16 hours, the vessel is taken therefrom and dried at 120 °C. Furthermore, an obtained vessel is dipped in ethanol and allylcarboxylic acid is added thereto, a solution of chloroplatinic acid in ethanol is added thereto and stirring is conducted at 60 °C for 2 hours. After reaction is completed, washing with ethanol and drying under a reduced pressure are conducted.

A material for composing a vessel is not particularly limited and it is possible to provide a styrene - divinylbenzene copolymer, a mica, a talc, a kaolin, an alumina, a titanium oxide, a zinc oxide, iron oxide, or the like.

An alkene that has a carboxyl group is not limited to allylcarboxylic acid and it is sufficient to be a carboxylic acid that has a vinyl group, a carboxylic acid that has an acryl group, or the like.

A method for introducing an amino group, a carboxyl group, and a hydroxyl group near a surface of a vessel is not particularly limited and it is possible to provide a method for conducting plasma treatment under an atmosphere that includes oxygen gas or ammonia gas, or the like. Specifically, after a vessel is contained in a reaction vessel and an inside of the vessel is vacuated by a vacuum pump, oxygen gas and ammonia gas are introduced and glow discharge is conducted to generate low temperature plasma.

For a group that has a reactivity with a carboxyl group, it is possible to provide an amino group, a hydroxyl group, or the like, wherein an amino group is preferable because of a high reactivity.

For a group that has a reactivity with an amino group or a hydroxyl group, it is possible to provide a carboxyl group, an aldehyde group, or the like, wherein a carboxyl group is preferable because of a high reactivity.

Furthermore, it is preferable for a surface modifying agent to be such that a group that has a reactivity with a carboxyl group, an amino group, or a hydroxyl group bonds to a group represented by general formula (1) via a spacer. A spacer is not particularly limited and it is possible to provide a methylene group, an oxyethylene group, an alkylene group that has one or more amino groups, or the like.

Next, a surface modifying agent that has an amino group and a group represented by general formula (1) will be described.

A surface modifying agent that has an amino group and a group represented by general formula (1) is not particularly limited and it is possible to provide a compound disclosed in Japanese Patent Application Publication No. 2006-007203, Japanese Patent Application Publication No. 2006-007204, or Japanese Patent Application Publication No. 2008-174491, or the like.

A carboxyl group that is present near a surface of a vessel and an amino group that is possessed by a surface modifying agent form an amide linkage as condensation thereof is conducted by a general reaction. Specifically, after a solution of N-hydroxysuccinimide and 1-ethyl-3-(3-dimethylaminopropyl)carbodiimide is charged into a vessel and a carboxyl group that is present near a surface of the vessel is actively esterified, a surface modifying agent is charged into the vessel.

Next, a surface modifying agent that has a hydroxyl group and a group represented by general formula (1) will be described.

A surface modifying agent that has a hydroxyl group and a group represented by general formula (1) is not particularly limited and it is possible to provide L-α-glycerophosphorylcholine or the like.

A carboxyl group that is present near a surface of a vessel and a hydroxyl group that is possessed by a surface modifying agent form an ester linkage as condensation thereof is conducted by a general reaction. Specifically, a surface modifying agent with a hydroxyl group activated by using cyanogen bromide is charged into a vessel.

Next, a surface modifying agent that has a carboxyl group and a group represented by general formula (1) will be described.

A surface modifying agent that has a carboxyl group and a group represented by general formula (1) is not particularly limited and it is possible to provide a compound disclosed in Japanese Patent Application Publication No. 2006-011381 or the like.

An amino group that is present near a surface of a vessel and a carboxyl group that is possessed by a surface modifying agent form an amide linkage as condensation thereof is conducted by a general reaction. Specifically, a surface modifying agent with a carboxyl group that has been actively esterified by using a solution of N-hydroxysuccinimide and 1-ethyl-3-(3-dimethylaminopropyl)carbodiimide is charged into a vessel.

A hydroxyl group that is present near a surface of a vessel and a carboxyl group that is possessed by a surface modifying agent form an ester linkage as condensation thereof is conducted by a general reaction. Specifically, after a hydroxyl group that is present near a surface of a vessel is activated by using cyanogen bromide, a surface modifying agent is added thereto.

Next, a surface modifying agent that has an aldehyde group and a group represented by general formula (1) will be described.

A surface modifying agent that has an aldehyde group and a group represented by general formula (1) is not particularly limited and it is possible to provide a compound disclosed in Japanese Patent Application Publication No. 2006-011383 or the like.

An amino group that is present near a surface of a vessel and an aldehyde group that is possessed by a surface modifying agent form an imino linkage as condensation thereof is conducted by a general reaction. Specifically, after a surface modifying agent and methanol are charged into a vessel and left at room temperature for 6 hours, sodium cyanotrihydroborate is added thereto at 0 °C, and heating and stirring are conducted overnight. For a reaction solvent, it is possible to use a protic solvent such as water, ethanol, or 2-propanol, as well as methanol, wherein a rate of introduction tends to be high in a case where methanol is used.

A hydroxyl group that is present near a surface of a vessel and an aldehyde group that is possessed by a surface modifying agent form an acetal linkage as addition thereof is conducted by a general reaction. Specifically, after a surface modifying agent and methanol are charged into a vessel and left at room temperature for 6 hours, sodium cyanotrihydroborate is added thereto at 0 °C, and heating and stirring are conducted overnight. For a reaction solvent, it is possible to use a protic solvent such as water, ethanol, or 2-propanol, as well as methanol, wherein a rate of introduction tends to be high in a case where methanol is used.

Additionally, a surface modifying agent that has a group that has a reactivity with an aldehyde group and a group represented by general formula (1) may be reacted by using a vessel wherein an aldehyde group and an amino group and/or a hydroxyl group are present near a surface thereof, instead of a vessel wherein a carboxyl group, an amino group, and a hydroxyl group are present near a surface thereof.

A method for introducing an aldehyde group near a surface of a vessel is not particularly limited and it is possible to provide a method for reacting glutaraldehyde with a vessel wherein an amino group is present near a surface thereof, or the like.

For a group that has a reactivity with an aldehyde group, it is possible to provide an amino group, a hydroxyl group, or the like, wherein an amino group is preferable because of a high reactivity.

Furthermore, it is preferable for a surface modifying agent to be in such a manner that a group that has a reactivity with an aldehyde group bonds to a group represented by general formula (1) via a spacer. A spacer is not particularly limited, and it is possible to provide a methylene group, an oxyethylene group, or an alkylene group that has one or more amino groups.

Next, a surface modifying agent that has an amino group and a group represented by general formula (1) will be described.

A surface modifying agent that has an amino group and a group represented by general formula (1) is not particularly limited and it is possible to provide a compound disclosed in Japanese Patent Application Publication No. 2006-007203, Japanese Patent Application Publication No. 2006-007204, or Japanese Patent Application Publication No. 2008-174491, or the like.

An aldehyde group that is present near a surface of a vessel and an amino group that is possessed by a surface modifying agent form an imino linkage as condensation thereof is conducted by a general reaction. Specifically, after a surface modifying agent and methanol are charged into a vessel and left at room temperature for 6 hours, sodium cyanotrihydroborate is added thereto at 0 °C, and heating and stirring are conducted overnight. For a reaction solvent, it is possible to use a protic solvent such as water, ethanol, or 2-propanol as well as methanol, wherein a rate of introduction tends to be high in a case where methanol is used.

Next, a surface modifying agent that has a hydroxyl group and a group represented by general formula (1) will be described.

A surface modifying agent that has a hydroxyl group and a group represented by general formula (1) is not particularly limited and it is possible to provide L-α-glycrophosphorylcholine or the like.

An aldehyde group that is present near a surface of a vessel and a hydroxyl group that is possessed by a surface modifying agent form an acetal linkage as addition thereof is conducted by a general reaction. Specifically, after a surface modifying agent and methanol are charged into a vessel and left at room temperature for 6 hours, sodium cyanotrihydroborate is added thereto at 0 °C, and heating and stirring are conducted overnight. For a reaction solvent, it is possible to use a protic solvent such as water, ethanol, or 2-propanol, as well as methanol, and a rate of introduction tends to be high in a case where methanol is used.

Furthermore, a surface modifying agent that has a group that has a reactivity with an amino group or a hydroxyl group and a group represented by general formula (1) may be reacted by using a vessel wherein an amino group or a hydroxyl group and a carboxyl group are present near a surface thereof, instead of a vessel wherein a carboxyl group, an amino group, and a hydroxyl group are present near a surface thereof.

Moreover, a surface modifying agent that has a group that has a reactivity with an amino group or a hydroxyl group and a phosphorylcholine-like group may be reacted by using a vessel wherein an amino group and a hydroxyl group are present near a surface thereof, instead of a vessel wherein a carboxyl group, an amino group, and a hydroxyl group are present near a surface thereof, in such a manner that an unreacted amino group and/or hydroxyl group remain(s) near a surface of the vessel.

Furthermore, a surface modifying agent that has a group that has a reactivity with a carboxyl group, an amino group, or a hydroxyl group and a group represented by general formula (1) may be reacted by using a vessel wherein a carboxyl group, an amino group, or a hydroxyl group are present near a surface thereof, instead of a vessel wherein a carboxyl group, an amino group, and a hydroxyl group are present near a surface thereof, in such a manner that an unreacted carboxyl group, amino group, or hydroxyl group remains near a surface of the vessel.

### [Second embodiment of the culturing vessel D]

A second embodiment of the culturing vessel D is manufactured by reacting a first surface modifying agent that has a group capable of producing a silanol group through hydrolysis thereof and a group represented by general formula (1) and a second surface modifying agent that has an amino group, a carboxyl group, and/or a hydroxyl group, and a group capable of producing a silanol group through hydrolysis thereof, with a vessel wherein a group capable of producing a silanol group through hydrolysis thereof, a silanol group, a hydroxyl group originating from a semimetal oxide, and/or a hydroxyl group originating from a metal oxide is/are present near a surface thereof. Herein, a molecular weight of a first surface modifying agent is 315 - 650. Thereby, it is possible to introduce a group represented by general formula (1) at a high density.

Additionally, when the adhesive cell C is a mesenchymal stem cell, it is preferable for a molar ratio of an amino group, carboxyl group, and/or hydroxyl group that is/are possessed by the second surface modifying agent to a group represented by general formula (1) that is possessed by the first surface modifying agent to be 0.001 - 2.4. If the molar ratio is less than 0.001, a cell may not adhere thereto, and if it is greater than 2.4, a cell proliferates on a condition of a monolayer, so that aggregation may not be attained.

For a method for introducing a group capable of producing a silanol group through hydrolysis thereof and/or a silanol group near a surface of a vessel, it is possible to provide a method for applying an application fluid that contains a polymer that has a group capable of producing a silanol group through hydrolysis thereof (which will be referred to as a "hydrolyzable polymer", hereinafter) and an alkoxysilane to a vessel.

As an application fluid that contains a hydrolysable polymer and an alkoxysilane is applied to a vessel, the hydrolyzable polymer and the alkoxysilane are hydrolyzed to produce a silanol group. Furthermore, a polymerizable polymer is crosslinked due to mutual dehydration and condensation of silanol groups so that a cross-linked polymer layer with an introduced silanol group is formed. Specifically, after an application fluid is applied to a material, water, an acid, or an alkali is applied thereto or heating thereof is conducted. Furthermore, after water, an acid, or an alikali is applied to a material, an application fluid may be applied thereto. Furthermore, water, an acid, or an alkali may be mixed to an application fluid. In such a case, it is preferable to prepare an application fluid appropriately at time of application, because hydrolysis occurs in an application fluid. Additionally, when water, an acid, or an alkali is used, reaction sufficiently proceeds at room temperature usually, although heating thereof may be conducted. Furthermore, reaction gently proceeds due to moisture in air, even though water, an acid, or an alkali is not used.

An acid or alikali that is used for hydrolysis is not particularly limited as long as it is possible to cause hydrolysis, wherein it is possible to mix two or more kinds thereof, and it may be used in an aqueous solution.

It is possible to use an application fluid wherein a polymerizable polymer and an alkoxysilane that are dissolved or dispersed in an organic solvent. An organic solvent is not particularly limited and it is possible to provide an aliphatic hydrocarbon, an aromatic hydrocarbon, a chlorinated hydrocarbon, an ether, an alcohol such as a 1 - 4-hydric aliphatic alcohol with a carbon number of 1 - 4, a cellosolve such as ethyl cellosolve or butylcellosolve, a dioxane, methyl acetate, diformamide, or the like, wherein two or more kinds thereof may be used in combination.

A content of a hydrolyzable polymer in an application fluid is usually 0.001 - 20% by mass, wherein 0.1 - 5% by mass is preferable. As a content of a hydrolyzable polymer in an application fluid is less than 0.001% by mass, it may not be possible to obtain sufficient effect by a single application, and if it is greater than 20% by mass, an application property may be degraded.

Furthermore, a mass ratio of a hydrolyzable polymer to an alkoxysilane is usually 0.01 - 20%, wherein 0.2 - 5% is preferable. If a mass ratio of a hydrolyzable polymer to an alkoxysilane is less than 0.01%, an intensity of a cross-linked polymer layer may be insufficient, and if it is greater than 20%, an amount of a silanol group(s) introduced into a cross-linked polymer may be insufficient.

A method for applying an application fluid is not particularly limited and it is possible to provide a dip coating method, a spray coating method, a spin cast method, or the like.

A material for composing a vessel is not particularly limited, and it is possible to provide an organic material such as a PP (poly(propylene)), a cycloolefin resin, a polycarbonate, a PET (poly(ethyleneterephthalte)), a PEEK, a fluororesin, a poly(styrene), or a poly(vinyl chloride); an inorganic material such as a gold, a titanium, an aluminum, an iron, a copper, a stainless steal, an alumina, a titanium oxide, a zinc oxide, or the like.

A hydrolyzable polymer is not particularly limited as long as a polymer has a group capable of producing a silanol group through hydrolysis thereof, and it is possible to use a homopolymer or copolymer obtained by polymerizing a monomer (A-1) represented by general formula of 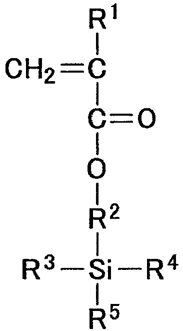 (in the formula, R¹ is a hydrogen atom or a methyl group, R² is an alkylene group with a carbon number of 1 - 6, and preferably, a propylene group, and each of R³, R⁴, and R⁵ is independently an alkoxy group with a carbon number of 1 - 6, and preferably a methoxy group or an ethoxy group.) (which will be referred to as polymer (A), hereinafter). Then, two or more kinds of monomers (A-1) may be used.

Furthermore, when polymer (A) is synthesized, monomer (A-2) represented by a general formula of 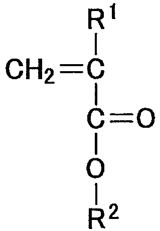 (in the formula, R¹ is a hydrogen atom or a methyl group, and R² is a linear, branched, or cyclic alkyl group with a carbon number of 1 - 18, and preferably, an alkyl group with a carbon number of 1 - 6, and particularly preferably, a methyl group.) may be copolymerized therewith. Then, two or more kinds of monomers (A-2) may be used.

Furthermore, when polymer (A) is synthesized, monomer (A-3) represented by a general formula of 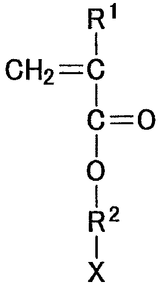 (in the formula, R¹ is a hydrogen atom or a methyl group, R² is an alkylene group with a carbon number of 1 - 6, and preferably an ethylene group, a propylene group, or a 2-hydroxypropylene group, and X is group (X-1) represented by a general formula of 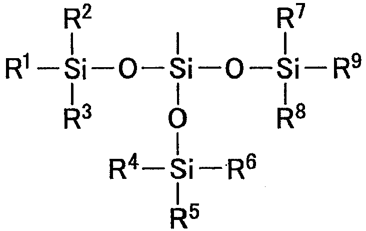 (in the formula, each of R¹, R², R³, R⁴, R⁵, R⁶, R⁷, R⁸, and R⁹ is independently a linear or branched alkyl group with a carbon number of 1 - 6, and preferably a methyl group.), group (X-2) represented by a general formula of 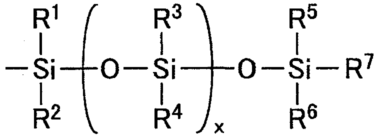 (in the formula, each of R¹, R², R³, R⁴, R⁵, and R⁶ is independently a linear or branched alkyl group with a carbon number of 1 - 6, and preferably a methyl group, R⁷ is a linear or branched alkyl group with a carbon number of 1 - 6, and preferably a butyl group, and x is a positive integer.), or group (X-3) represented by a general formula of 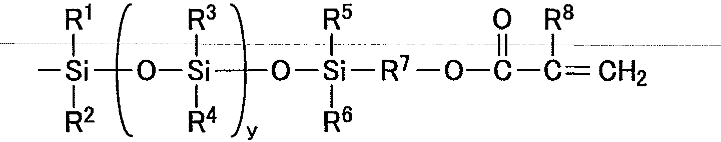 (in the formula, each of R¹, R², R³, R⁴, R⁵, and R⁶ is independently a linear or branched alkyl group with a carbon number of 1 - 6, and preferably a methyl group, R⁷ is an alkylene group with a carbon number of 1 - 6, and preferably an ethylene group, a propylene group, or a 2-hydroxypropylene group, R⁸ is a hydrogen atom or a methyl group, and y is a positive integer.)) may be copolymerized therewith. Additionally, when X is group (X-2) or (X-3), a molecular weight of monomer (A-3) is preferably 1000 - 100000, and particularly preferably 2000 - 20000. Herein, two or more kinds of monomers (A-3) may be used.

Furthermore, when polymer (A) is synthesized, monomer (A-4) represented by a general formula of 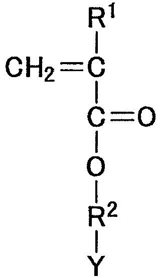 (in the formula, R¹ is a hydrogen atom or a methyl group, R² is an alkylene group with a carbon number of 1 - 6, and preferably an ethylene group or a propylene group, and Y is group (Y-1) represented by a general formula of 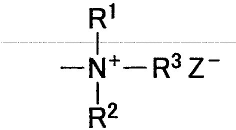 (in the formula, each of R¹, R², and R³ is independently an alkyl group with a carbon number of 1 - 6, and preferably a methyl group, and Z⁻ is a halide ion or a conjugate ion of an organic acid or inorganic acid.) or group (Y-2) represented by a general formula of 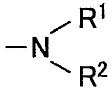 (in the formula, each of R¹ and R² is independently an alkyl group with a carbon number of 1 - 6, and preferably a methyl group.)) may be copolymerized therewith. Herein, two or more kinds of monomers (A-4) may be used.

That is, when polymer (A) is synthesized, monomer (A-2), monomer (A-3), and/or monomer (A-4) may be copolymerized with monomer (A-1).

A content of monomer (A-1) in all monomers used for synthesizing polymer (A) is preferably 40 - 85% by mass. If a content of monomer (A-1) in all monomers is less than 40% by mass, a crosslink density may be degraded so that an effect of hydrophilization may not sufficiently retained, and if it is greater than 85% by mass, a uniformity of a cross-linked polymer layer may be degraded.

Furthermore, a content of monomer (A-2) in all monomers to be used for synthesizing polymer (A) is usually 1 - 75% by mass, and preferably 10 - 60% by mass. If a content of monomer (A-2) in all monomers is less than 1% by mass, a water resistance of a cross-linked polymer layer may be degraded, and if it is greater than 75% by mass, polymer (A) may be difficult to be dissolved in an alcohol.

Furthermore, a content of monomer (A-3) in all monomers to be used for synthesizing polymer (A) is usually 1 - 70% by mass, and preferably 5 - 60% by mass. If a content of monomer (A-3) in all monomers is less than 1% by mass, a water resistance of a cross-linked polymer layer may be degraded, and if it is greater than 70% by mass, polymer (A) may be difficult to be dissolved in an alcohol.

Furthermore, a ratio of a mass of monomer (A-4) to a total mass of monomer (A-1), monomer (A-2), and monomer (A-3) is usually 0.01 - 1, and preferably 0.05 - 0.5. If this ratio is less than 0.01, a flexibility of a cross-linked polymer layer may be degraded, and if it is greater than 1, a water resistance of a cross-linked polymer layer may be degraded.

A number average molecular weight of polymer (A) is preferably 2000 - 150000. If a number average molecular weight of polymer (A) is less than 2000, a period of time for forming a cross-linked polymer layer may be long, and if it is greater than 150000, a viscosity of an application fluid may be high so that an application property or a working property may be degraded.

Additionally, a specific example of and a manufacturing method for polymer (A) are disclosed in Japanese Patent Application Publication No. 11-302129 or the like.

Furthermore, for a hydrolyzable polymer, it is possible to use a homopolymer or copolymer that has a structural unit (B-1) represented by 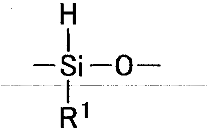 (in the formula, R1 is an alkyl group with a carbon number of 1 - 22 or phenyl group, and preferably a methyl group.) (which will be referred to as polymer (B), hereinafter). Herein, polymer (B) may have two or more kinds of structural units (B-1).

Furthermore, polymer (B) may have a structural unit (B-2) represented by a general formula of 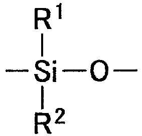 (in the formula, each of R¹ and R² is independently an alkyl group with a carbon number of 1 - 22 or a phenyl group, and preferably a methyl group.). Herein, polymer (B) may have two or more kinds of structural units (B-2).

For polymer (B), a content of structural unit (B-1) is preferably 1 - 90% by mass. If a content of structural unit (B-1) is less than 1% by mass, a crosslink density may be degraded so that an effect of hydrophilization may not sufficiently be retained, and if it is greater than 90% by mass, a uniformity of a cross-linked polymer layer may be degraded.

Furthermore, for polymer (B), a content of structural unit (B-2) is preferably 10 - 99% by mass. If a content of structural unit (B-2) is less than 10% by mass, a uniformity of a cross-linked polymer layer may be degraded, and if it is greater than 99% by mass, a crosslink density may be degraded so that an effect of hydrophilization may not sufficiently be retained.

A number average molecular weight of polymer (B) is preferably 2000 - 500000. If a number average molecular weight is less than 2000, a period of time for forming a cross-linked polymer layer may be long, and if it is greater than 500000, a viscosity of application fluid may be high so that an application property or a working property may be degraded.

For a hydrolyzable polymer, polymer (A) and polymer (B) may be used in combination, and a hydrolyzable polymer and a non-hydrolyzable polymer may be used in combination. A non-hydrolyzable polymer is not particularly limited and it is possible to provide polymer (A) or polymer (B) that does not have a group capable of producing a silanol group through hydrolysis thereof or the like.

A method for introducing a silanol group near a surface of a vessel is not particularly limited and it is possible to provide a method for forming a film that includes a silicone resin that has a silanol group, by applying an application fluid that includes a silicone resin to a vessel, or the like.

For a film that includes a silicone resin that has a silanol group, a contact angle of water is preferably 3 - 8°. It is difficult to form a film with a contact angle of water that is less than 3°, and if a film with a contact angle of water that is greater than 8° is formed, it may be impossible to introduce a group represented by general formula (1) at a high density. A silicone resin to be included in an application fluid is not particularly limited, and it is possible to provide a resin obtained by hydrolyzing and then condensing an alkoxysilane represented by a general formula of

(R¹O)ₙSi(R²)₄₋ₙ

(in the formula, each of R¹ and R² is independently an alkyl group with a carbon number of 1 - 8 and n is an integer of 1 - 4, wherein when n is 1 or 2, a plurality of R²s may be identical or different, and when n is 2 or 3, a plurality of R¹s may be identical or different.), wherein two or more kinds thereof may be in combination. Herein, a silicone resin that has a silanol group to be included in a film with a contact angle of water that is 3 - 8° may be identical to or different from a silicone resin to be included in an application fluid.

An organic solvent to be included in an application fluid is not particularly limited, and it is possible to provide an aliphatic hydrocarbon, an aromatic hydrocarbon, a chlorinated hydrocarbon, an ether, an alcohol such as a 1 - 4-hydric aliphatic alcohol with a carbon number of 1 - 4, a cellosolve such as ethylellosolve or butylcellosolve, a dioxane, methyl acetate, a diformamide, or the like, wherein two or more kinds thereof may be in combination.

A content of a silicone resin in an application fluid is usually 0.001 - 1% by mass, and preferably 0.1 - 1% by mass. If a content of a silicone resin in an application fluid is less than 0.001% by mass, a uniform film may not be formed, and if it is greater than 20% by mass, an application property may be degraded.

A method for applying an application fluid is not particularly limited, and it is possible to provide a dip coating method, a spray coating method, a spin-cast method, or the like.

A material for composing a vessel is not particularly limited, and it is possible to provide an organic material such as a polycarbonate, a PET (poly(ethyleneterephthalate)), a poly(styrene), or an acryl resin; an inorganic material such as a gold, a titanium, an aluminum, an iron, a copper, a stainless steel, an alumina, a titanium oxide, or a zinc oxide; or the like.

Furthermore, a vessel made of a glass may be used for a vessel wherein a silanol group is present near a surface thereof.

A metal oxide for composing a vessel wherein a hydroxyl group originating from a metal oxide is present near a surface thereof is not particularly limited, and it is possible to provide a titanium oxide, a zinc oxide, an iron oxide, a chromium oxide, an aluminum oxide, or the like.

A metal oxide for composing a vessel wherein a hydroxyl group originating from a semimetal oxide is present near a surface thereof is not particularly limited, and it is possible to provide a germanium oxide, an arsenic oxide, a boron oxide, or the like.

For a group that has a reactivity with a group capable of producing a silanol group through hydrolysis thereof, a silanol group, a hydroxyl group originating from a semimetal oxide, and/or a hydroxyl group originating from a metal oxide, it is possible to provide a group capable of producing a silanol group through hydrolysis thereof.

Furthermore, for a first surface modifying agent, a group capable of producing a silanol group through hydrolysis thereof preferably bonds to a group represented by general formula (1) via a spacer. A spacer is not particularly limited and it is possible to provide a methylene group, an oxyethylene group, an alkylene group that has one or more amino groups, or the like.

For a group capable of producing a silanol group through hydrolysis thereof, it is possible to provide a hydrosilyl group, an alkoxysilyl group, a halosilyl group, an acyloxysilyl group, an amino silyl group, or the like, wherein an alkoxysilyl group with a carbon number of 1 - 6 or a hydrosilyl group is preferable from the viewpoint of stability, reactivity, or the like.

A first surface modifying agent is not particularly limited as long as a group capable of producing a silanol group through hydrilysis thereof and a group represented by general formula (1) are possessed, and it is possible to provide a compound disclosed in Japanese Patent Application Publication No. 2006-011380.

A second surface modifying agent is not particularly limited as long as an amino group, a group capable of producing a carboxyl group through hydrolysis thereof, and/or a group capable of producing a hydroxyl group through hydrolysis thereof, and a group capable of producing a silanol group through hydrolysis thereof are possessed, and it is possible to provide a compound that has an amino group, such as 3-aminopropyltrimethoxysilane or p-aminophenyltrimethoxysilane; a compound that has a group capable of producing a carboxyl group through hydrolysis thereof, such as 3-(triethoxysilyl)propylsuccinic anhydride; a compound that has a group capable of producing a hydroxyl group through hydrolysis thereof, such as 3-glycidoxypropyltrimethoxysilane or 3-glycidoxypropylmethyldiethoxysilane, or the like, wherein two or more kinds thereof may be used in combination.

When a second surface modifying agent has a group capable of producing a carboxyl group and/or a hydroxyl group through hydrolysis thereof, it is sufficient to introduce a group capable of producing a carboxyl group and/or a hydroxyl group through hydrolysis thereof near a surface of a vessel, and subsequently, produce a carboxyl group and/or a hydroxyl group through hydrolysis thereof.

Additionally, an amino group that is possessed by a second surface modifying agent may be protected with a protective group. Furthermore, a second surface modifying agent may have a carboxyl group or hydroxyl group that is protected by a protective group capable of deprotection by a reaction other than hydrolysis thereof, instead of a group capable of producing a carboxyl group or a hydroxyl group through hydrolysis thereof.

When a second surface modifying agent has an amino group, carboxyl group and/or hydroxyl group that is protected by a protective group(s), it is sufficient to introduce an amino group, carboxyl group and/or hydroxyl group that is protected by a protective group near a surface of a vessel and subsequently produce an amino group, a carboxyl group, and/or a hydroxyl group due to deprotection.

Next, a method will be described for introducing an amino group, a group capable of producing a carboxyl group through hydrolysis thereof and/or a group capable of producing a hydroxyl group through hydrolyis thereof, and a group represented by general formula (1) to a vessel wherein a group capable of producing a silanol group through hydrolysis thereof, a silanol group, a hydroxyl group originating from a semimetal oxide, and/or a hydroxyl group originating from a metal oxide is/are present near a surface thereof.

First, an application fluid that includes a first surface modifying agent and a second surface modifying agent is applied to a vessel wherein a group capable of producing a silanol group through hydrolysis thereof, a silanol group, a hydroxyl group originating from a semimetal oxide, and/or a hydroxyl group originating from a metal oxide is/are present near a surface thereof. Herein, a group capable of producing a silanol group through hydrolysis thereof is hydrolyzed to produce a silanol group. Moreover, a surface of a vessel is modified by dehydration and condensation of a silanol group that is present near a surface of a vessel, a hydroxyl group produced by hydrolysis of a semimetal oxide, and/or a hydroxyl group produced through hydrolysis of a metal oxide, and a silanol group produced through hydrolysis of a surface modifying agent. Specifically, after an application fluid is applied to a vessel, water, an acid, or an alkali may be applied thereto or heating thereof may be heated. Furthermore, an application fluid may be applied after water, an acid, or an alkali is applied to a vessel. Moreover, water, an acid, or an alkali may be mixed into an application fluid. In this case, it is preferable to prepare an application fluid appropriately at a time of application, because hydrolysis occurs in the application fluid. Additionally, when water, an acid, or an alkali is used, a reaction usually proceeds sufficiently at room temperature although heating may be conducted. Furthermore, a reaction gently proceeds due to moisture in an atmosphere, even though water, an acid, or an alkali is not used.

An acid or an alkali is not particularly limited as long as it is possible to hydrolyze a group capable of producing a silanol group through hydrolysis thereof, wherein two or more kinds thereof may be used in combination. Additionally, an acid or alkali to be used for hydrolysis may be used in an aqueous solution.

It is possible to use an application fluid wherein a first surface modifying agent and a second surface modifying agent are dissolved or dispersed in an organic solvent. For an organic solvent, it is possible to provide an aliphatic hydrocarbon, an aromatic hydrocarbon, a chlorinated hydrocarbon, an ether, an alcohol such as a 1 - 4-hydric aliphatic alcohol with a carbon number of 1 - 4, a cellosolve such as ethylcellosolve or butylcellosolve, a dioxane, methyl acetate, diformamide, or the like.

A content of a first surface modifying agent in an application fluid is usually 0.1 - 30% by mass and preferably 1 - 10% by mass. If a content of a first surface modifying agent in an application fluid is less than 0.1% by mass, it may be impossible to apply the first surface modifying agent sufficiently by a single application, and if it is greater than 30% by mass, an application property may be degraded.

A method for applying an application fluid is not particularly limited, and it is possible to provide a dip coating method, a spray coating method, a spin-cast method, or the like.

Additionally, each of an application fluid that includes a first surface treating agent and an application fluid that includes a second surface treating agent may be applied to a vessel.

Furthermore, a silanol group originating from a vessel, a hydroxyl group originating from a semimetal oxide, and/or a hydroxyl group originating from a metal oxide may be reacted with a silanol group originating from a surface modifying agent, so that an unreacted silanol group, a hydroxyl group originating from a semimetal oxide, and/or a hydroxyl group originating from a metal oxide remain(s) near a surface of the vessel.

### [Third embodiment of the culturing vessel D]

In a third embodiment of the culturing vessel D, a layer that includes a resin that has an amino group, a carboxyl group, and/or a hydroxyl group and a group represented by general formula (1) is formed on a surface thereof.

A resin that has an amino group, a carboxyl group, and/or a hydroxyl group and a group represented by general formula (1) is not particularly limited, and it is possible to provide a resin obtained by copolymerizing a monomer that has a group represented by general formula (1) and a monomer that has an amino group, a monomer that has a carboxyl group, and/or a monomer that has a hydroxyl group (see, for example, Japanese Patent Application Publication No. 7-184990).

A material for composing a vessel is not particularly limited, and it is possible to provide an organic material such as a cycloolefin resin, a polycarbonate, a PET (poly(ethylene terephthalate)), a poly(styrene), or an acryl resin; an inorganic material such as a gold, a titanium, an aluminum, an iron, a copper, a stainless steel, an alumina, a titanium oxide, or zinc oxide; or the like.

Additionally, when a vessel composed of an organic material is used, it is preferable to use a resin obtained by copolymerizing a monomer that has a group represented by general formula (1), a monomer that has an amino group, a carboxyl group, and/or a hydroxyl group, and a monomer that has a hydrophobic group, by taking an adhesive property to a vessel into consideration.

A monomer that has a hydrophobic group is not particularly limited and it is possible to provide butyl methacrylate or the like.

For a method for forming a layer that includes a resin that has an amino group, a carboxyl group, and/or a hydroxyl group, and a group represented by general formula (1) near a surface of a vessel, it is possible to provide a method for applying to a vessel an application fluid that includes a resin that has an amino group, a carboxyl group, and/or a hydroxyl group, and a group represented by general formula (1).

It is possible to use an application fluid wherein a resin is dissolved or dispersed in an organic solvent. For an organic solvent, it is possible to provide an aliphatic hydrocarbon, an aromatic hydrocarbon, a chlorinated hydrocarbon, an ether, an alcohol such as a 1 - 4-hydric aliphatic alcohol with a carbon number of 1 - 4, a cellosolve such as ethylcellosolve or butylcellosolve, a dioxane, methyl acetate, diformamide, or the like.

A content of a resin in an application fluid is usually 0.1 - 30% by mass, and preferably 1 - 10% by mass. If a content of a resin in an application fluid is less than 0.1% by mass, it may be impossible to apply a resin sufficiently by a single application, and if it is greater than 30% by mass, an application property may be degraded.

A method for applying an application fluid is not particularly limited and it is possible to provide a dip coating method, a spray coating method, a spin-case method, or the like.

As an adhesive cell originating from a mammal such as a human being, a rat, a mouse, or a primate is applied to a method for forming a cell aggregate in the present invention, it is useful for a method for screening a substance and a method for exploring a function of a cell as described below.

In the present invention, a method for culturing an adhesive cell is not particularly limited, and it is possible to apply a publicly known condition depending on an adhesive cell, wherein it is possible to provide a method for culturing an adhesive cell at 37 °C under an atmosphere that includes 5% by volume of carbon dioxide, by using a Dulbecco's modified medium (DMEM) that includes 10% by mass of a fetal bovine serum (FBS), 1 mM of glutamine, and a suitable amount of an antibiotic substance, or the like.

In a method for screening a substance in the present invention, a substance is screened by using a cell aggregated that is formed by a method for forming a cell aggregate in the present invention. Herein, it is possible to apply a method for screening a substance in the present invention to a publicly known method for screening a substance such as a method for evaluating a toxicity of a substance by using a hepatic cell or a myocardial cell or a method for testing an effect of a drug by using a cancer cell. For a method for evaluating a toxicity of a substance, it is possible to provide a method based on a cell proliferation such as an MTT assay or an Alamar Blue array. Furthermore, it is also possible to quantify an amount of SDF-1, LIF, EGFR, or the like that is a factor associated with production, releasing, or canceration of an inflammatory cytokine, by a method such as RT-PCR or ELISA.

Furthermore, in a method for screening a substance in the present invention, it is possible to screen a substance by using a cell aggregate of adhesive cells for each individual so that it is possible to be applied to a tailor-made medicine.

In a method for exploring a function of a cell in the present invention, a function of a cell is explored by using a cell aggregate that is formed by a method for forming a cell aggregate in the present invention. Herein, it is possible to apply a method for exploring a function of a cell in the present invention to a publicly known method for exploring a function of a cell such as a method for elucidating a biochemical function of an adhesive cell or a method for exploring a biomarker.

### PRACTICAL EXAMPLES

### [Practical Example 1]

After a 1% by mass aqueous solution of 1 g of N-hydroxysuccinimide and 1 g of 1-ethyl-3-(3-dimethylaminopropyl)carbodiimide was charged into a Primaria dish (produced by Becton Dickinson and Company), a solution wherein 1 g of compound (A) represented by a chemical formula of 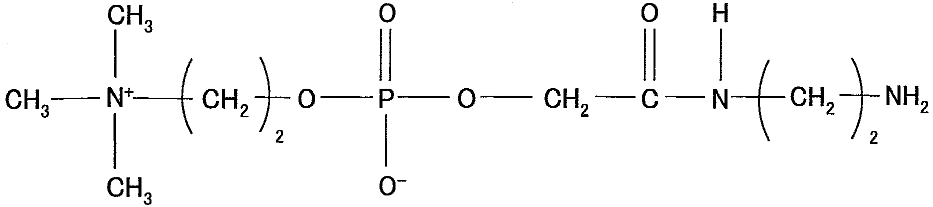 is dissolved in 100 ml of water was added thereto, so that amidation was conducted. Then, after a fluid was taken from the Primaria dish and dried at room temperature for 5 hours, washing with water and drying were conducted to obtain a culturing vessel. Additionally, the Primaria dish was a dish made of a poly(styrene) plasma-treated under an atmosphere that included oxygen and ammonia.

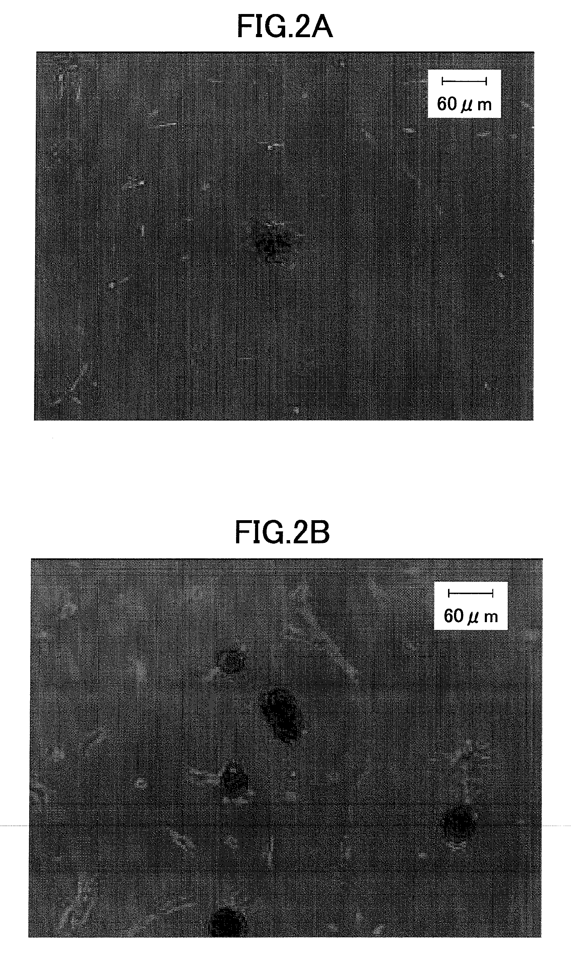

As 10 ml of a Mensen PRO RS medium (produced by Invitrogen Corporation) and 1 × 10⁶ / vessel of stem cells originating from a human fat (produced by Invitrogen Corporation) as mesenchymal stem cells were added to the culturing vessel and the stem cells originating from a human fat were cultured, the cells proliferated and autonomously aggregated to form a cell aggregate (see FIG. 2A and FIG. 2B).

### [Comparative Example 1]

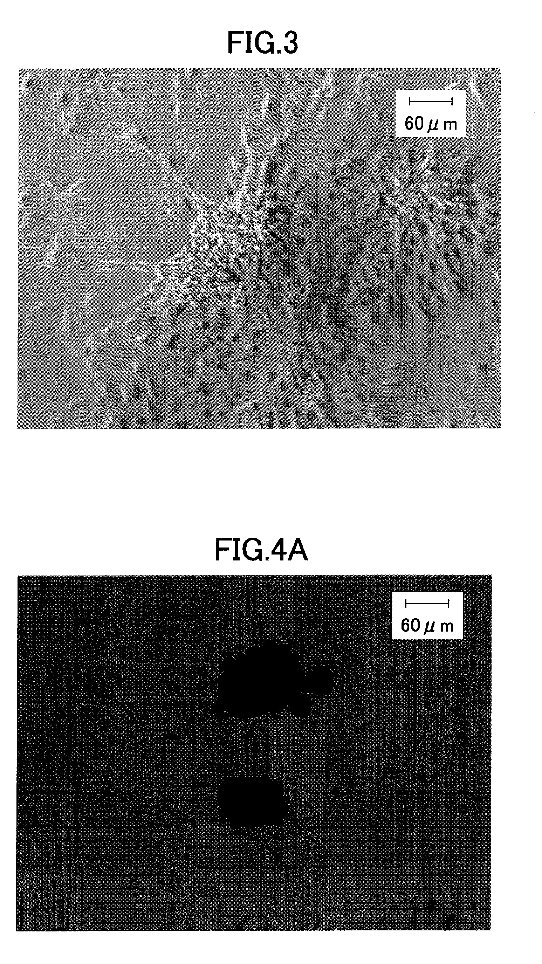

As 10 ml of a Mensen PRO RS medium (produced by Invitrogen Corporation) and 1 × 10⁶ / dish of stem cells originating from a human fat (produced by Invitrogen Corporation) as mesenchymal stem cells were added to a Petri dish made of a poly(styrene) with an untreated surface (produced by Becton Dickinson and Company) and the stem cells originating from a human fat were cultured, the cells proliferated on a condition of a monolayer and a cell aggregate was not formed (see FIG. 3).

### [Alkaline phosphatase activity]

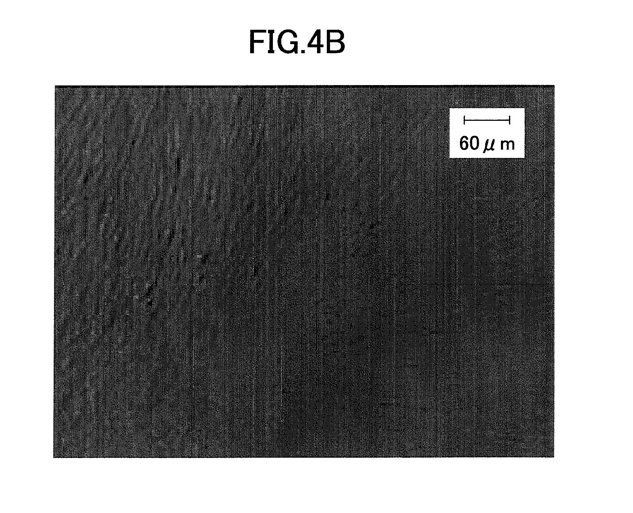

After 4% by mass of a paraformaldehyde phosphate buffer solution was added to the cell aggregate in Practical Example 1 and the cultured cells in Comparative Example 1 and treatment was conducted for 5 minutes, washing was conducted by using a TBS buffer solution and a TBST buffer solution. Then, after a BM purple AP substrate (produced by F. Hoffmann-La Roche Ltd.) was added and a reaction was conducted at 37 °C for 1 hour, washing was conducted by using a TBST buffer solution. Results of evaluation were illustrated in FIG. 4A and FIG. 4B. It could be found from FIG. 4A and FIG. 4B that the cell aggregate in Practical Example 1 was positive for an alkaline phosphatase activity thereof and cultured cells in Comparative example 1 was negative for an alkaline phosphatase activity thereof.

### [Immune double staining]

After 4% by mass of a paraformaldehyde phosphate buffer solution was added to the cell aggregate in Practical Example 1 and the cultured cells in Comparative Example 1 and treatment was conducted for 5 minutes, washing was conducted by using a TBS buffer solution and a TBST buffer solution. Then, after a first primary antibody was diluted by a method described in a catalogue thereof and reacted with cells, a first Alexa Fluor-labeled secondary antibody (produced by Invitrogen Corporation) was reacted with the cells and washing was conducted by using a TBST buffer solution. Moreover, after a second primary antibody was diluted by a method described in a catalogue thereof and reacted with the cells, a second Alexa Fluor-labeled secondary antibody (produced by Invitrogen Corporation) was reacted with the cells and washing was conducted by using a TBST buffer solution. Results of evaluation were illustrated in Table 1.

**Table 1**

| | Practical Example 1 | Comparative Example 1 |
|---|---|---|
| Nestin | Positive | Negative |
| CD271 | High | Low |
| CD133 | Positive | Negative |
| PDGFRa | High | Low |
| PDGFRb | High | Low |

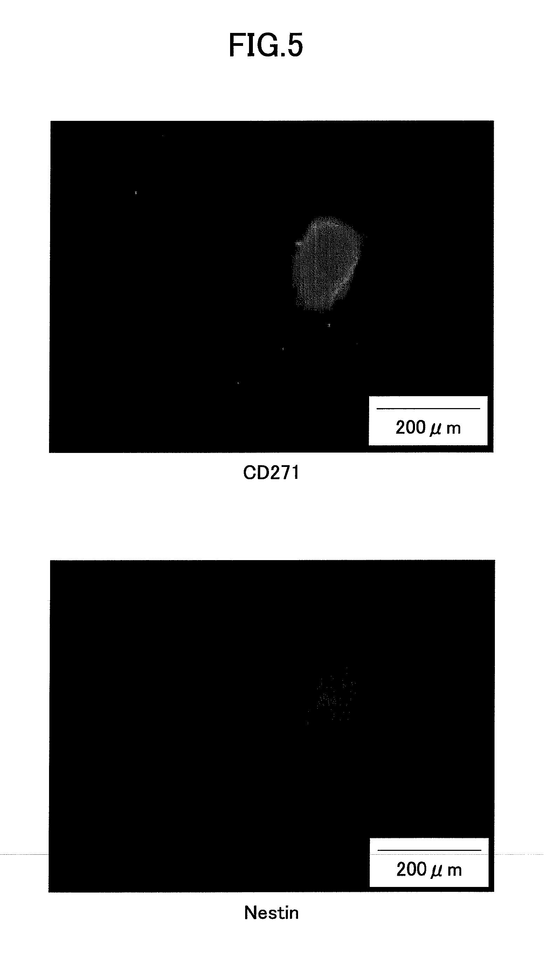

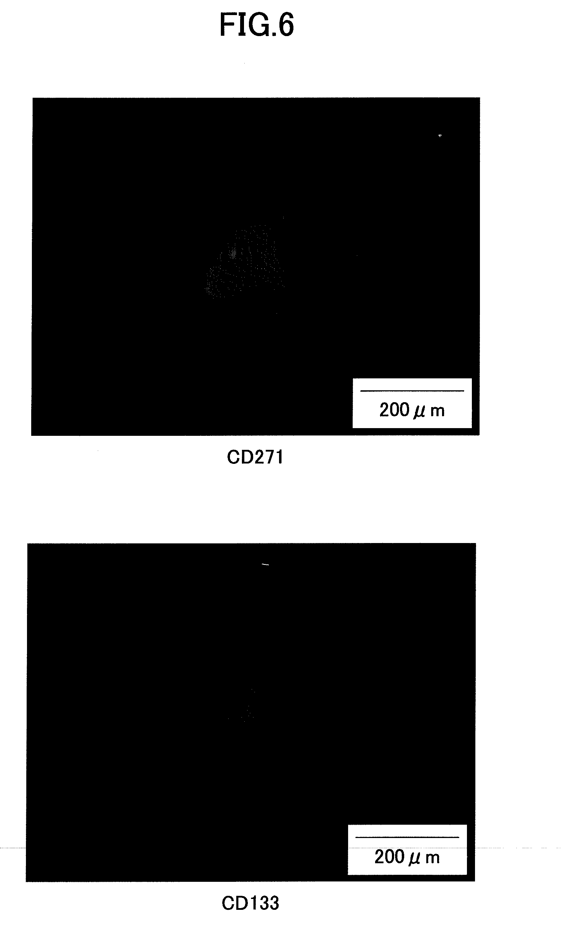

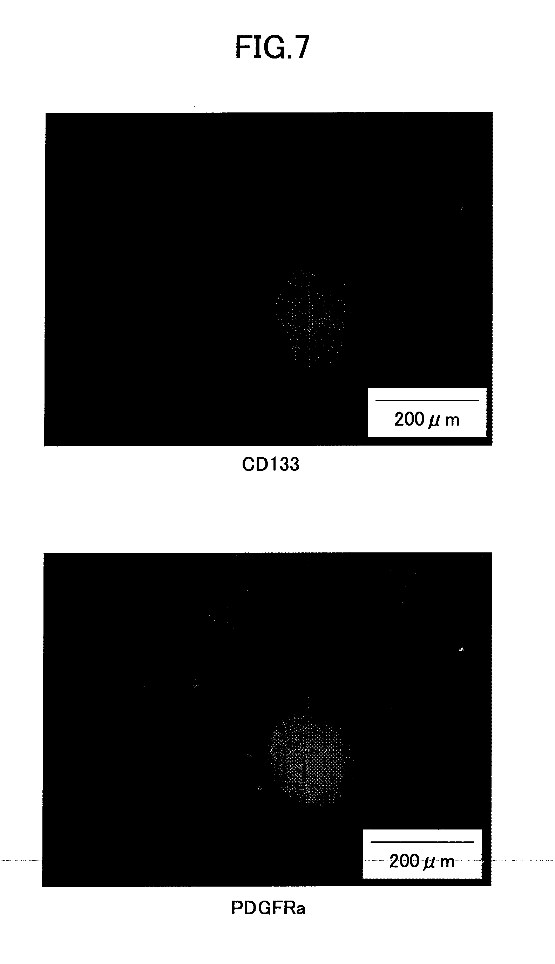

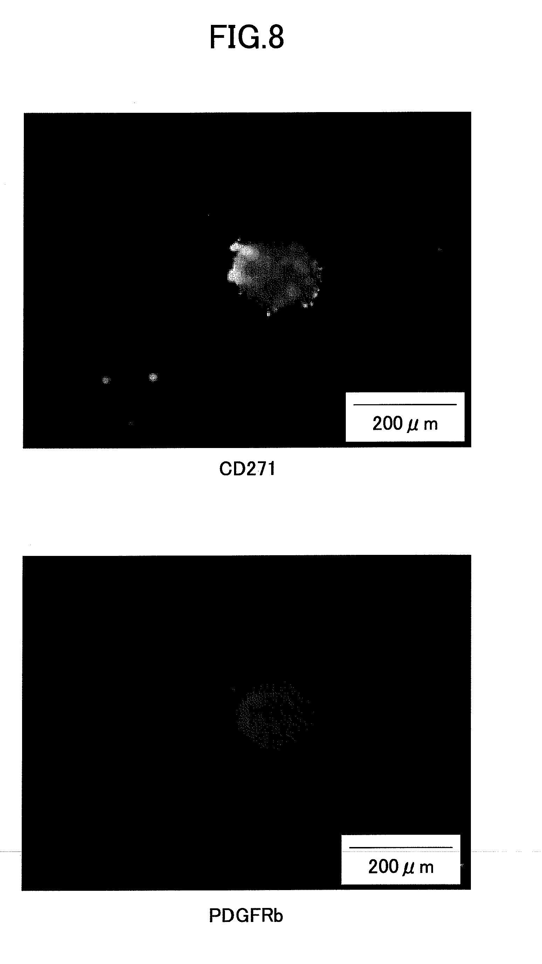

Additionally, Nestin (produced by Abcam plc.), CD271 (produced by Abcam plc.), and CD133 (produced by Abcam plc.) were a marker for a stem cell originating from a nerve, a marker for a stem cell originating from a fat, and a marker for a stem cell, respectively, and PDGFRa (produced by R&D Systems, Inc.) and PDGFRb (produced by R&D Systems, Inc.) were markers for mesenchymal stem cells. Furthermore, results of evaluation of the cell aggregate in Practical Example 1 are illustrated in FIGS. 5 - 8. It could be found that it was possible for a stem cell originating from a fat to acquire a property of a stem cell originating from a nerve in vitro, because the cell aggregate in Practical Example 1 was positive for Nestin from Table 1.

### [Practical Example 2-1]

An application fluid was obtained that was composed of 0.01 mL of 0.15 mol/L solution of 3-aminopropyltrimethoxysilane in methanol, 9.99 mL of 0.15 mol/L solution of compound (B) represented by a chemical formula of 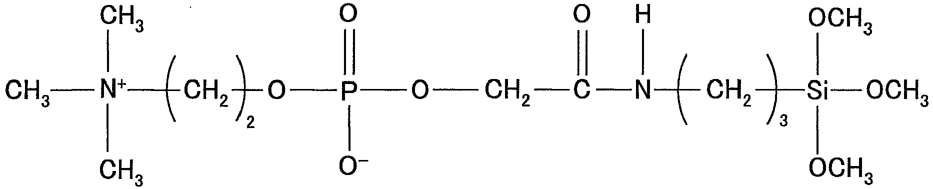 in methanol, and 10 mL of methanol.

An obtained application fluid was applied to a Glass Bottom Dish (produced by Matsunami Glass Ind., Ltd.) and dried at room temperature. Then, after drying by heating and drying at room temperature were conducted, washing with water and drying were conducted to obtain a culturing vessel.

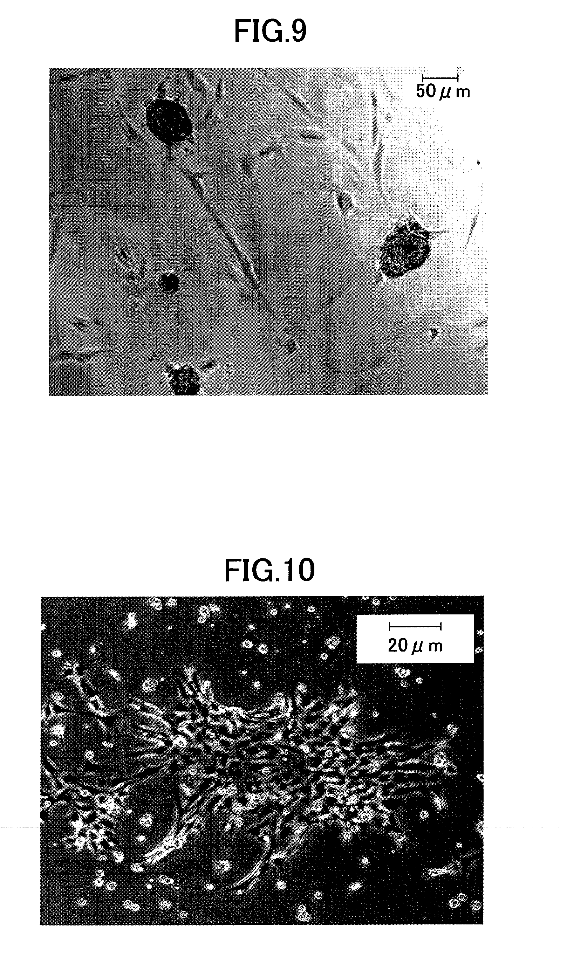

As 2.5 ml of a Mensen PRO RS medium (produced by Invitrogen Corporation) and 1 × 10⁵ / vessel of stem cells originating from a human fat (produced by Invitrogen Corporation) were added to the culturing vessel and the stem cells originating from a human fat were cultured, the cells proliferated and autonomously aggregated to form a cell aggregate (see FIG. 9).

### [Practical Example 2-2]

An application fluid was obtained that was composed of 0.5 mL of a 0.15 mol/L solution of 3-aminopropyltrimethoxysilane in methanol, 9.5 mL of a 0.15 mol/L solution of compound (B) in methanol, and 10 mL of methanol.

An obtained application fluid was applied to a Glass Bottom Dish (produced by Matsunami Glass Ind., Ltd.) and dried at room temperature. Then, after drying by heating and drying at room temperature were conducted, washing with water and drying were conducted to obtain a culturing vessel.

As 2.5 ml of a Mensen PRO RS medium (produced by Invitrogen Corporation) and 1 × 10⁵ / vessel of stem cells originating from a human fat (produced by Invitrogen Corporation) were added to the culturing vessel and the stem cells originating from a human fat were cultured, the cells proliferated and autonomously aggregated to form a cell aggregate (see FIG. 10).

### [Practical Example 2-3]

An application fluid was obtained that was composed of 7 mL of a 0.15 mol/L solution of 3-aminopropyltrimethoxysilane in methanol, 3 mL of a 0.15 mol/L solution of compound (B) in methanol, and 10 mL of methanol.

An obtained application fluid was applied to a Glass Bottom Dish (produced by Matsunami Glass Ind., Ltd.) and dried at room temperature. Then, after drying by heating and drying at room temperature were conducted, washing with water and drying were conducted to obtain a culturing vessel.

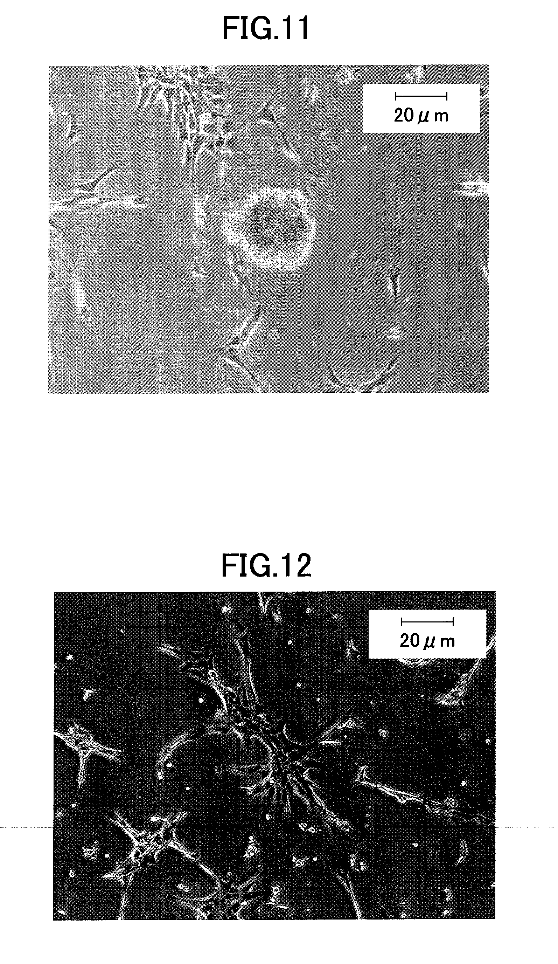

As 2.5 ml of a Mensen PRO RS medium (produced by Invitrogen Corporation) and 1 × 10⁵ / vessel of stem cells originating from a human fat (produced by Invitrogen Corporation) were added to the culturing vessel and the stem cells originating from a human fat were cultured, the cells proliferated and autonomously aggregated to form a cell aggregate (see FIG. 11).

### [Comparative Example 2]

As 2.5 ml of a Mensen PRO RS medium (produced by Invitrogen Corporation) and 1 × 10⁵ / dish of stem cells originating from a human fat (produced by Invitrogen Corporation) were added to a Glass Bottom Dish with an untreated surface (produced by Matsunami Glass Ind., Ltd.) and the stem cells originating from a human fat were cultured, the cells proliferated on a condition of a monolayer and a cell aggregate was not formed (see FIG. 12).

### [Immunostaining]

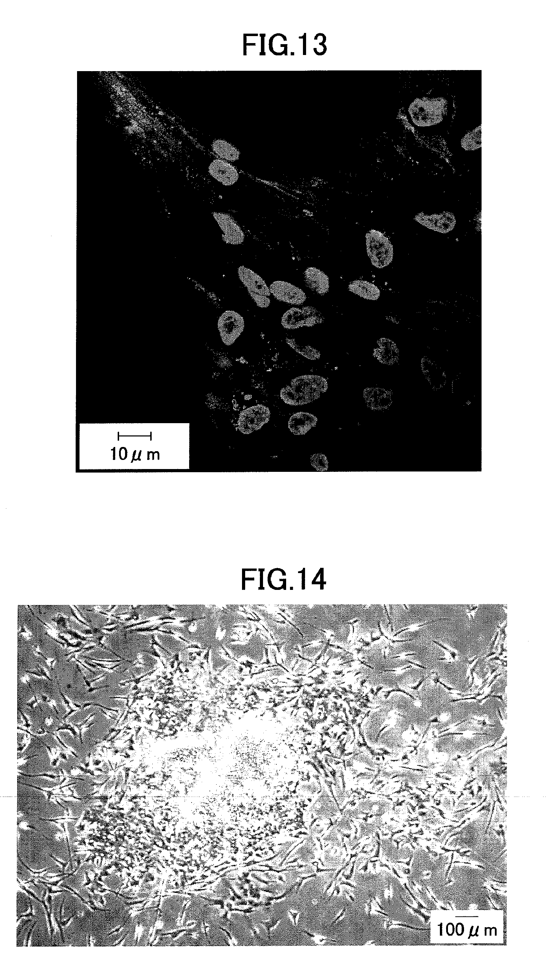

After 4% by mass of paraformaldehyde phosphate buffer solution was added to the cell aggregate in Practical Example 2-2 to conduct treatment for 5 minutes, washing was conducted by using 0.2% by mass of Triton X-100 (produced by Roche Diagnostics K. K.) / TBS buffer solution and 0.2% by mass of Tween 20 (produced by Tokyo Chemical Industry Co., Ltd.) / TBS buffer solution. Then, a nonspecific reaction was blocked by Protein Block (produced by DAKO). Moreover, after anti-CD 271 rabbit polyclonal antibody (produced by Abcam plc.) and CD 90 mouse monoclonal antibody (produced by Abcam plc.) as primary antibodies diluted 200 times were reacted with the cells overnight, washing was conducted by using 0.2% by mass of Tween 20 (produced by Tokyo Chemical Industry Co., Ltd.) / TBS buffer solution. Then, after Alexa 488 anti-rabbit antibody (produced by Invitrogen Corporation) and Alexa 594 anti-mouse antibody (produced by Invitrogen Corporation) as fluorescent secondary antibodies that were diluted 250 times and developed a green color and a red color, respectively, were reacted with the cells for 2 hours, washing was conducted by using 0.2% by mass of Tween 20 (produced by Tokyo Chemical Industry Co., Ltd.) / TBS buffer solution. Moreover, after nuclei were stained with Vectorshield-DAPI (produced by VECTOR LABORATORIES, INC.) that developed a blue color, observation was conducted by using a confocal laser microscope LSM 5 PASCAL (produced by Carl Zeiss). As a result, it was found that CD 271 was positive, because the cell aggregate in Practical Example 2 - 2 developed a green color (see FIG. 13).

Additionally, the cell aggregates in Practical Example 2 - 1 and Practical Example 2 - 3 also developed green colors similarly to the cell aggregate in Practical Example 2 - 2, so that CD271 was positive.

### [Practical Example 3]

After an aqueous solution wherein 89 mg of N-hydroxysuccinimide and 149 mg of 1-ethyl-3-(3-dimethylaminopropyl)carbodiimide were dissolved in 10 mL of water was charged into a Primaria dish (produced by Becton Dickinson and Company), a solution wherein 200 mg of compound (A) was dissolved in 10 mL of water was added thereto, so that amidation was conducted. Then, after a fluid was taken from the Primaria dish, washing with water and drying were conducted to obtain a culturing vessel.

As 3 ml of an MEM medium (produced by SIGMA-ALDRICH CO.) that includes 10% by mass of a fetal bovine serum (FBS) and 1 × 10⁵ / vessel of human hepatic cancer cells (produced by Primary Cell Co., Ltd.) as cancer cells were added to the culturing vessel and the human hepatic cancer cells were cultured, the cells proliferated and autonomously aggregated to form a cell aggregate (see FIG. 14).

### [Comparative Example 3]

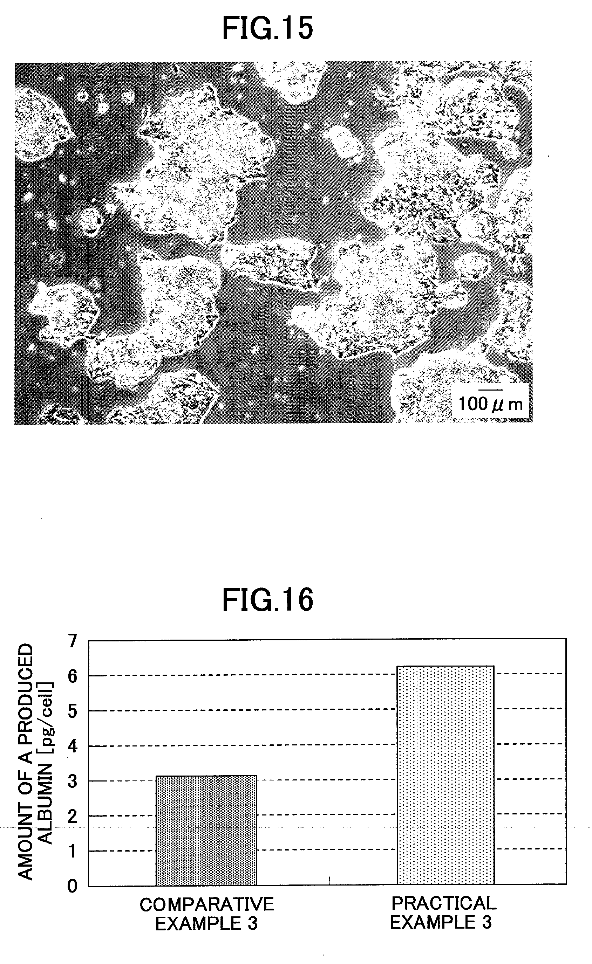

As 3 ml of an MEM medium (produced by SIGMA-ALDRICH CO.) that includes 10% by mass of a fetal bovine serum (FBS) and 1 × 10⁵ / vessel of human hepatic cancer cells (produced by Primary Cell Co., Ltd.) as cancer cells were added to a Primaria dish (produced by Becton Dickinson and Company) and the human hepatic cancer cells were cultured, the cells proliferated on a condition of a monolayer and a cell aggregate was not formed (see FIG. 15).

### [Amount of albumin production]

Amounts of albumin production in media wherein the human hepatic cancer cells in Practical Example 3 and Comparative Example 3 were cultured were quantified by using a human albumin quantification kit (produced by COSMO BIO co., ltd.) in accordance with an ELISA method. Results of evaluation are illustrated in FIG. 16. It could be found from FIG. 16 that an amount of albumin production of the cell aggregate in Practical Example 3 was larger than that of the cultured cells in Comparative Example 3.

### [Activity of CYP1A1]

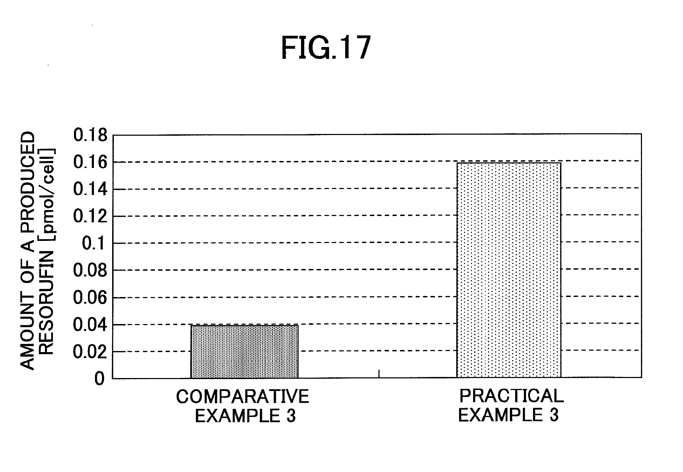

The cell aggregate in Practical Example 3 and the cultured cells in Comparative Example 3 were cultured in an MEM medium (produced by SIGMA-ALDRICH CO.) that included 10% by mass of a fetal bovine serum (FBS), 8 µM of 7-ethoxyresorufin and 10 µM of dicoumarol at 37 °C for 30 minutes. Then, an acetic acid buffer solution that includes β-glucuronidase and arylsulfatase was added to a culturing supernatant, and reaction was conducted at 37 °C for 2 hours. Moreover, an amount of resorufin in the solution was quantified by a fluorescence measurement (excitation wavelength: 530 nm, and fluorescence wavelength: 590 nm), so that an activity of CYP1A1 produced by the cells was evaluated. Results of evaluation are illustrated in FIG. 17. It could be found from FIG. 17 that an activity of CYP1A1 for the cell aggregate in Practical Example 3 was greater than that for the cultured cells in Comparative Example 3.

### [Gene expression]

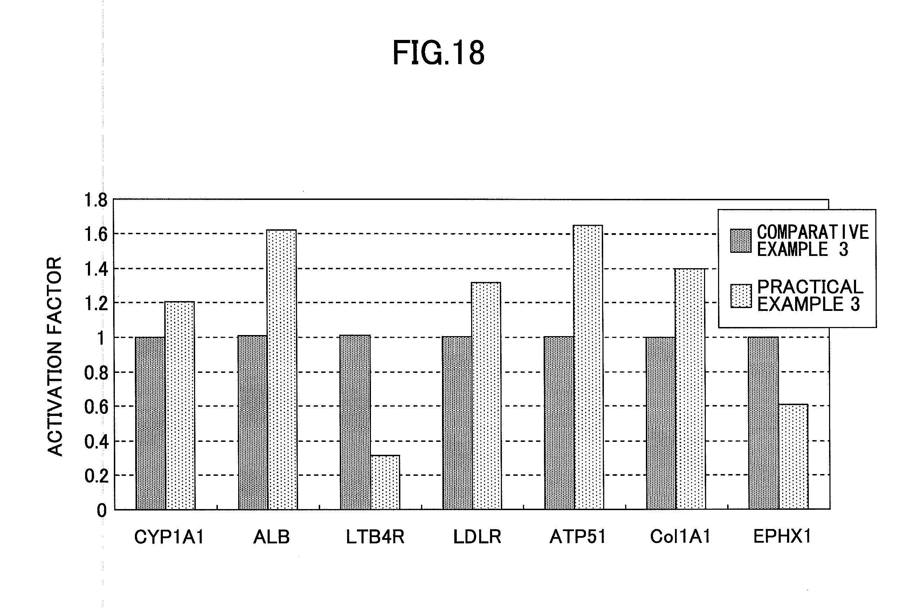

Gene expressions of the cell aggregate in Practical Example 3 and the cultured cells in Comparative Example 3 were quantified by using Lightcycler 480-II (produced by F. Hoffmann-La Roche Ltd.) in accordance with a PCR method. Results of evaluation are illustrated in FIG. 18. It could be found from FIG. 18 that gene expressions of CYP1A1, ALB, LDLR, ATP51, and Col1A1 for the cell aggregate in Practical Example 3 were greater than those for the cultured cells in Comparative Example 3. Additionally, GAPDH is used for an internal standard.

### [Practical Example 4]

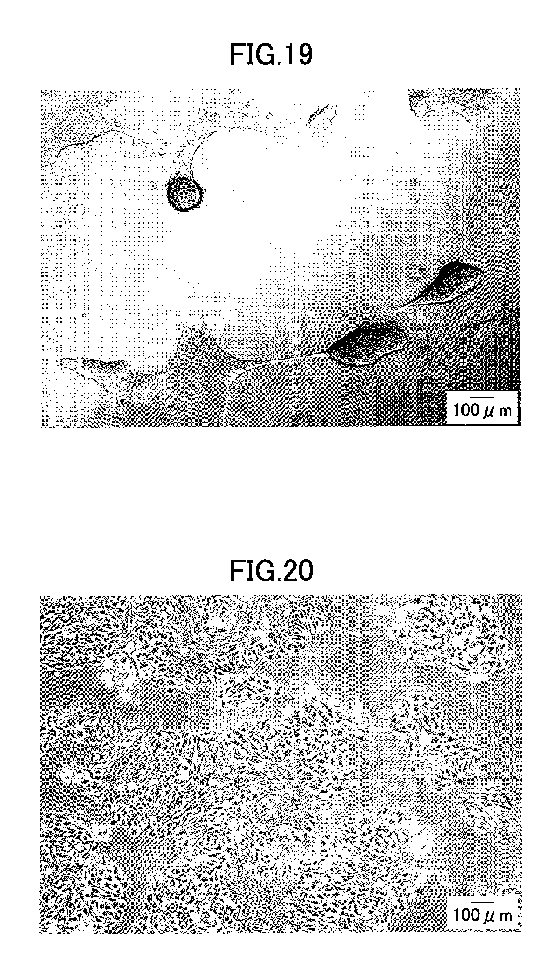

As cancer cells originating from a human mammary gland were cultured similarly to Practical Example 3 except that cancer cells originating from a human mammary gland (produced by RIKEN BRC) was used instead of the human hepatic cancer cells (produced by Primary Cell Co., Ltd.), the cells proliferated and autonomously aggregated to form a cell aggregate (see FIG. 19).

### [Comparative Example 4]

As cancer cells originating from a human mammary gland were cultured similarly to Comparative Example 3 except that cancer cells originating from a human mammary gland (produced by RIKEN BRC) was used instead of the human hepatic cancer cells (produced by Primary Cell Co., Ltd.), the cells proliferated on a condition of a monolayer and a cell aggregate was not formed (see FIG. 20).

### [Alkaline phosphatase activity]

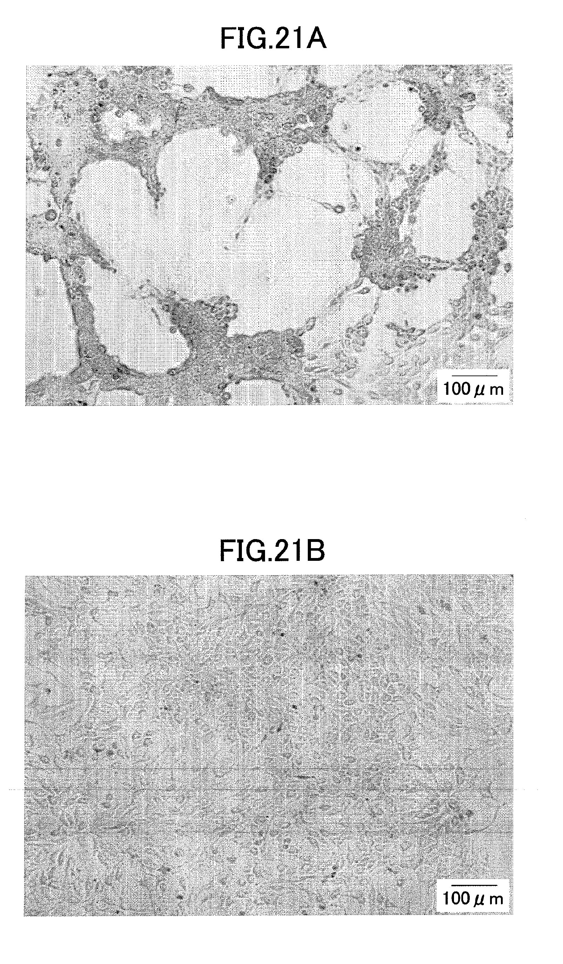

After 4% by mass of a paraformaldehyde phosphate buffer solution was added to the cell aggregate in Practical Example 4 and the cultured cells in Comparative Example 4 and treatment was conducted for 5 minutes, washing was conducted by using a TBS buffer solution and a TBST buffer solution. Then, after a BM purple AP substrate (produced by F. Hoffmann-La Roche Ltd.) was added and a reaction was conducted at 37 °C for 1 hour, washing was conducted by using a TBST buffer solution. Results of evaluation were illustrated in FIG. 21A and FIG. 21B. It could be found from FIG. 21A and FIG. 21B that the cell aggregate in Practical Example 4 was positive for an alkaline phosphatase activity thereof and cultured cells in Comparative example 4 was negative for an alkaline phosphatase activity thereof.

### [Gene expression]

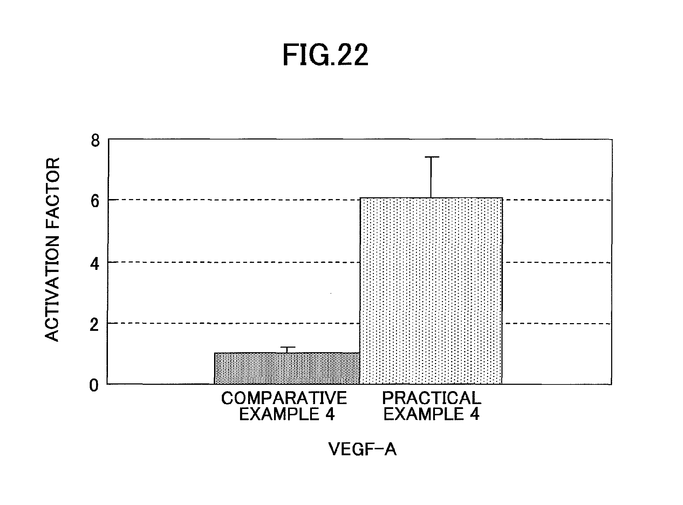

Gene expressions of the cell aggregate in Practical Example 4 and the cultured cells in Comparative Example 4 were quantified by using Lightcycler 480-II (produced by F. Hoffmann-La Roche Ltd.) in accordance with a PCR method. Results of evaluation are illustrated in FIG. 22. It could be found from FIG. 22 that gene expression of VEGF-A for the cell aggregate in Practical Example 4 was greater than those for the cultured cells in Comparative Example 4. Additionally, GAPDH is used for an internal standard.

The present international application claims priority based on Japanese Patent Application No. 2010-277394 filed on December 13, 2010 and Japanese Patent Application No. 2011-265210 filed on December 2, 2011, and the entire contents of Japanese Patent Application No. 2010-277394 and Japanese Patent Application No. 2011-265210 are incorporated by reference in the present international application.

### EXPLANATION OF LETTERS OR NUMERALS

C: ADHESIVE CELL
C': CELL AGGREGATE
D: CULTURING VESSEL
M: MEDIUM

## Claims

1. A method for forming a cell aggregate by using a culturing vessel and culturing an adhesive cell, **characterized in that** the culturing vessel is such that an amino group, a carboxyl group, and/or a hydroxyl group, and a group represented by a general formula of 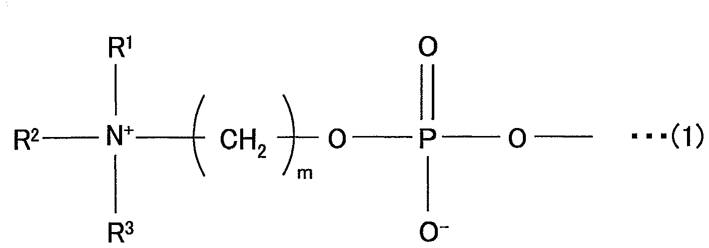 (in the formula, each of R¹, R², and R³ is independently an alkyl group with a carbon number greater than or equal to 1 and less than or equal to 6 and m is an integer greater than or equal to 2 and less than or equal to 6.) are present near a surface thereof and the adhesive cell is a mesenchymal stem cell, a hepatic cell, or a cancer cell.

2. The method for forming a cell aggregate as claimed in claim 1, **characterized in that** the culturing vessel is manufactured by reacting a compound that has a group that has a reactivity with a carboxyl group, an amino group, or a hydroxyl group and a group represented by the general formula and a molecular weight that is greater than or equal to 225 and less than or equal to 650, with a vessel with a carboxyl group, an amino group, and a hydroxyl group that are present near a surface thereof.

3. The method for forming a cell aggregate as claimed in claim 2, **characterized in that** the vessel with a carboxyl group, an amino group, and a hydroxyl group that are present near a surface thereof is manufactured by conducting a plasma treatment in an atmosphere that includes oxygen, ozone, and/or water vapor and ammonia and/or nitrogen.

4. The method for forming a cell aggregate as claimed in claim 1, **characterized in that** the culturing vessel is manufactured by reacting a first compound that has a group capable of producing a silanol group through hydrolysis thereof and a group represented by the general formula and a molecular weight that is greater than or equal to 315 and less than or equal to 650 and a second compound that has an amino group, a group capable of producing a carboxyl group through hydrolysis thereof, and/or a group capable of producing a hydroxyl group through hydrolysis thereof, and a group capable of producing a silanol group through hydrolysis thereof, with a vessel with a group capable of producing a silanol group through hydrolysis thereof, a silanol group, a hydroxyl group originating from a semimetal oxide and/or a hydroxyl group originating from a metal oxide that is/are present near a surface thereof.

5. The method for forming a cell aggregate as claimed in claim 1, **characterized in that** the culturing vessel is such that a layer that includes a resin that has an amino group, a carboxyl group, and/or a hydroxyl group and a group represented by the general formula is formed on a surface thereof.

6. A method for screening a substance, **characterized by** having a step of forming a cell aggregate by using the method for forming a cell aggregate as claimed in any one of claims 1 to 5 and a step of screening a substance by using the cell aggregate.

7. A method for exploring a function of a cell, **characterized by** having a step of forming a cell aggregate by using the method for forming a cell aggregate as claimed in any one of claims 1 to 5 and a step of exploring a function of a cell by using the cell aggregate.

8. A cell aggregate wherein an adhesive cell is proliferated to aggregate autonomously, **characterized in that** the adhesive cell is a mesenchymal stem cell, a hepatic cell, or a cancer cell.

9. A culturing vessel, **characterized in that** an amino group, a carboxyl group, and/or a hydroxyl group, and a group represented by a general formula of 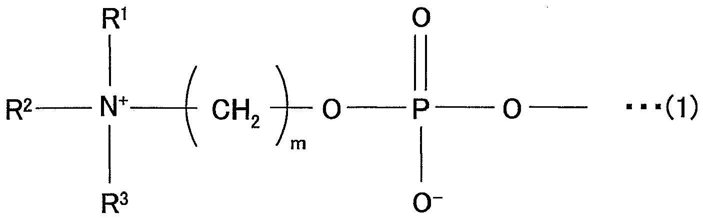 (in the formula, each of R¹, R², and R³ is independently an alkyl group with a carbon number greater than or equal to 1 and less than or equal to 6 and m is an integer greater than or equal to 2 and less than or equal to 6.) are present near a surface thereof.
